# EUROPÄISCHE PATENTANMELDUNG

(11) **EP 0 634 373 A1**
(43) Veröffentlichungstag der Anmeldung: **18.01.1995**
(21) Anmeldenummer: 94106893.4
(22) Anmeldetag: 03.05.1994
(51) Int. Cl.: C03C 3/091, C03C 4/00, A61K 6/06

(54) **Bariumfreies Dentalglas mit guter Röntgenabsorption**

(30) Priorität: 10.07.1993 DE 4323143
(71) Anmelder: Schott Glaswerke, D-55122 Mainz (DE); Carl-Zeiss-Stiftung trading as SCHOTT GLASWERKE, D-55122 Mainz (DE)
(72) Erfinder: Grabowski, Danuta, D-65232 Taunusstein (DE); Daimer, Johann, D-84051 Oberahrain (DE); Clement, Marc, Dr., D-55118 Mainz (DE); Paschke, Hartmut, Dr., D-84030 Ergolding (DE)

(57) **Zusammenfassung**

Es wird ein bariumfreies Dentalglas mit guter Röntgenabsorption beschrieben, das eine Zusammensetzung in Gew.-% auf Oxidbasis von SiO₂ 45-65, B₂O₃ 5-20, Al₂O₃ 5-20, CaO 0-10, SrO 15-35, und F₂-O 0-2 besitzt. Das Glas besitzt einen Brechungsindex von ≦ 1.56 und eine Al-Gleichwertdicke von ≧ 3 mm und einen Wärmeausdehnungskoeffizienten α im Bereich von - 30 bis + 70 von ≦ 5 x 10⁻⁶ /K. Das Glas besitzt sehr gute mechanische und chemische Eigenschaften und kann nach der Vermahlung auf in der Dentaltechnik übliche Korngrößen in Dentalkompositen für als Zahnfüllung geeignete Kunstharzpasten verwendet werden.

## Beschreibung

Für Zahnfüllungen werden in zunehmenden Maß Dental-Komposite eingesetzt, um mögliche Nebenwirkungen der Amalgamfüllungen zu umgehen. Dental-Komposite bestehen in der Regel aus einem anorganischen Anteil und einem organischen Kunstharz-Binder. Der anorganische Anteil wird dabei aus einem Glaspulver hergestellt. An das verwendete Glaspulver werden neben den für eine gute Füllung notwendigen Pulvereigenschaften bestimmte Anforderungen an die physikalischen Eigenschaften des für das Pulver zu verwendenden Glases gestellt.

Das Glas soll einen dem Brechungsindex des Kunstharz-Binders angepaßten Brechungsindex besitzen. Durch diese Brechwertanpassung wird sichergestellt, daß die Füllung vom Zahnschmelz kaum noch zu unterscheiden ist.

Weiterhin wichtig ist, daß die Füllungen im Röntgenbild gut erkennbar sind. Damit wird sichergestellt, daß diese durch die Brechwertanpassung weitgehend unsichtbaren Füllungen vom Zahnarzt im Röntgenbild auch als Füllungen erkannt werden können.

Weiterhin soll der Wärmeausdehnungskoeffizient des Glases in einer Größenordnung liegen, die auch der des Zahnschmelzes besitzt. Weicht nämlich der Wärmeausdehnungskoeffizient des anorganischen Füllmaterials zu stark von dem des Zahnschmelzes ab, so besteht die Gefahr, daß die Füllung durch die thermischen Belastungen, denen sie im oralen Bereich durch unterschiedlich heiße Speisen ausgesetzt ist, an Haltbarkeit verliert.

Es ist bekannt, daß eine hohe Röntgenabsorption durch einen entsprechenden Gehalt von BaO erreicht werden kann. Da dieser Bestandteil jedoch an sich toxisch ist, sollte BaO als Bestandteil von für Füllungen benutzte Gläser aus rein prophylaktischen Gründen vermieden werden.

Die Aufgabe der Erfindung besteht daher darin, ein Dentalglas zu finden, das eine gute Röntgenabsorption besitzt und frei ist von Bariumoxid.

Diese Aufgabe wird durch das in Patentanspruch 1 beschriebene bariumfreie Dentalglas gelöst.

Das Glas ist aus einer minimalen Zahl von Komponenten aufgebaut. Diese minimale Zahl an Komponenten wurde deshalb gewählt, um die toxikologische Beurteilung des Glases auf möglichst wenige Komponenten begrenzen zu können.

Als Glasbildner werden die Komponenten SiO₂ und B₂O₃ benutzt. Dabei werden die Anteile an B₂O₃ zugegeben, um die Schmelztemperatur bei der Erschmelzung des Glases zu senken. Dies hat sowohl den Effekt, daß dadurch die Herstellung des Glases erleichtert wird, als auch daß durch die niedrigen Schmelztemperaturen sichergestellt wird, daß die Verunreinigungen, die durch die Auskleidung des Schmelzaggregates in das Glas gelangen können, auf ein Minimum verringert werden. SiO₂ ist in dem Glas in Mengen von 45 - 65 Gew.-% vorhanden. Bei Überschreiten von 65 Gew.-% steigt die Schmelztemperatur an, wodurch sowohl die Schmelztemperatur als auch die durch die Auskleidung des Schmelzaggregates in das Glas gelangenden Verunreinigungen ansteigen. Unterschreitet man die Untergrenze von 45 Gew.-%, so nimmt die hydrolytische Beständigkeit des Glases ab. Bevorzugt wird ein Gehalt von 48 - 60 Gew.-%, insbesondere von 50 - 56 Gew.-%, da sich in diesen Bereichen sowohl günstige Werte für die Schmelztemperatur als auch für die chemischen Eigenschaften dieses Glases ergeben. B₂O₃ ist in dem Glas in Mengen von 5 - 20 Gew.-%, bevorzugt 5 - 18,5 Gew.-%, insbesondere 6 - 15 Gew.-% vorhanden. Überschreitet man die Obergrenze von 20 Gew.-%, ergibt sich zwar eine ungewöhnlich niedrige Schmelztemperatur für das Glas, jedoch nimmt auch die hydrolytische Beständigkeit in unerwünschter Weise ab. Unterhalb von 5 Gew.-% nehmen die Schmelztemperatur und damit die aus dem Material des Schmelzaggregates kommenden Verunreinigungen immer mehr zu. In dem Bereich von 5 - 18,5 Gew.-% und insbesondere 6 - 15 Gew.-% für den B₂O₃-Anteil ergeben sich sowohl für die Herstellung des Glases als auch für seine Gebrauchseigenschaften besonders günstige Werte.

Als weiteren Glasbildner erhält das Glas Al₂O₃. Diese Komponente ist für die chemische Beständigkeit und für die Härte des Glases wesentlich. Dem Gehalt an Al₂O₃ sind allerdings Grenzen gesetzt, da ein steigender Al₂O₃-Gehalt im Glas die Schmelzbarkeit des Glases immer weiter verschlechtert, d. h. daß die verwendeten Prozeßtemperaturen immer weiter gesteigert werden müssen. Daher sollte ein Al₂O₃-Gehalt von 20 Gew.-% nicht überschritten werden. Ein hoher Gehalt an Al₂O₃ führt auch zu einer gesteigerten Neigung des Glases zur Entglasung, so daß die Gefahr besteht, daß nach dem Schmelzprozeß ein Glas erhalten wird, daß nach seiner Verarbeitung zu Pulver ein Pulver mit einer nicht homogenen Teilchenzusammensetzung ergibt. Unterschreitet man einen Anteil von 5 Gew.-% Al₂O₃, so können die Beschriebenen Vorteile des Al₂O₃ Gehaltes hinsichtlich Beständigkeit und Härte nicht mehr voll zur Geltung kommen. Sehr gute Ergebnisse sowohl hinsichtlich der Herstellbarkeit des Glases als auch hinsichtlich der Eigenschaften erhält man im Bereich von 5 - 18,5 und insbesondere 6 - 15 Gew.-% Al₂O₃.

Zur Erzielung einer ausreichenden Röntgenabsorption wird dem Glas ein Anteil von 15 - 35 Gew.-% an Strontiumoxid zugesetzt. Strontiumoxid besitzt ein ähnliches Röntgenabsorptionsverhalten wie Bariumoxid, aber nicht dessen Toxizität. In seinen toxikologischen Eigenschaften ähnelt es eher dem Calciumoxid, welches ebenfalls in Mengen von bis zu 10 Gew.-% vorhanden sein kann. Calciumoxid und Strontiumoxid sind an sich in dem Glas austauschbar, jedoch besitzt Calciumoxid ein gegenüber Strontiumoxid deutlich erniedrigtes Röntgenabsorptionsvermögen, so daß bei einer ausschließlichen Verwendung von Calciumoxid die geforderte Röntgenabsorption nicht erreicht werden kann. Es ist daher zur Erzielung einer ausreichenden Röntgenabsorption erforderlich, den Gehalt an SrO nicht unter 15 Gew.-% sinken zu lassen. Bevorzugt wird jedoch ein Mindestgehalt von Strontiumoxid von 17, insbesondere von 20 Gew.-%. Calciumoxid kann in dem Glas in Mengen von bis zu 10 Gew.-% vorhanden sein. Bevorzugt wird jedoch ein Calciumoxidgehalt von höchstens 7, insbesondere höchstens 6 Gew.-%. Sowohl Strontiumoxid, als auch Calciumoxid fördern in diesem Glassystem die Entglasung, so daß die Maximalmengen an SrO und CaO nicht überschritten werden sollten.

Falls erforderlich, kann das Glas noch bis zu 2 Gew.-% Fluor, gerechnet als F₂-O enthalten. Der Wert F₂-O gibt den Gewichtsanteil an, der durch den Ersatz von einem Sauerstoff-Ion durch zwei Fluor-Ionen erhalten wird.

Die Gläser enthalten keine weiteren Komponenten, insbesondere keine Alkalien. Letztere können zwar die Schmelzbarkeit des Glases verbessern, wirken jedoch negativ auf die hydrolytische Beständigkeit. Eine gute hydrolytische Beständigkeit ist jedoch wichtig, um Wechselwirkungen des Glases mit seiner Umgebung möglichst auszuschließen.

Die für das Glas verwendeten Rohstoffe sollen auf mögliche toxische Bestandteile sorgfältig kontrolliert werden, so daß mögliche Verunreinigungen an toxischen Bestandteilen unterhalb eines Wertes von insgesamt 20 ppm im Glas bleiben.

Für die zahnärztliche Praxis ist die gute Erkennbarkeit im Röntgenbild von hoher Bedeutung. Eine Aluminiumgleichwertdicke von ≧ 3 mm ist daher erforderlich und sollte nicht unterschritten werden. Bevorzugt wird eine Aluminiumgleichwertdicke von ≧ 4 mm. Unter Aluminiumgleichwertdicke wird die Dicke einer Aluminiumplatte verstanden, die die gleiche Röntgenabsorption wie eine 2 mm dicke Glasplatte hervorruft.

Für die Haltbarkeit der späteren Füllung ist ferner ein geeigneter Wärmeausdehnungskoeffizient α erforderlich. Dieser Wärmeausdehnungskoeffizient a soll im Bereich von - 30 bis + 70 °C einen Wert von 5 x 10⁻⁶ /K nicht überschreiten. Bevorzugt wird ein Wärmeausdehnungskoeffizient in diesem Bereich, der unter 4,5 x 10⁻⁶ /K liegt. Die erfindungsgemäß hergestellten Gläser besitzen diese erforderlichen physikalischen Eigenschaften und sind daher für die Verwendung in der Zahnrestauration besonders geeignet.

Nach seiner Herstellung wird aus dem Glas in an sich bekannter Weise z. B. durch Mahlen und ggf. Sieben ein Glaspulver hergestellt, das die für Dentalzwecke durchschnittliche Teilchengröße von < 10 µm, insbesondere 0,5 - 5 µm, bevorzugt von 0,7 - 1,5 µm besitzt. Zur Erzielung guter mechanischer Eigenschaften ist in üblicher Weise eine nicht zu enge Korngrößenverteilung günstig, wie sie beispielsweise durch übliche Vermahlung und Absiebung der Grobanteile erreicht wird. Jedoch sollte eine maximale Teilchengröße von 40 µm, vorzugsweise 20 µm insbesondere 10 µm nicht überschritten werden. In dieser Form ist das Glaspulver zur Verwendung als Füllmittel für als Zahnfüllmittel verwendete Dentalkomposite besonders geeignet.

Zur Herstellung von als Zahnfüllmittel verwendbaren Dentalkompositen wird das Glaspulver mit in der Zahnmedizin üblichen härtbaren Kunstharzen gemischt. Als Kunstharz werden überwiegend UV-härtbare Harze auf Acrylat-, Methacrylat-, Bis-GMA- (2,2-Bis-[4-(3-methacryloxi-2-hydroxipropoxi)-phenyl]-propan), Urethan-Methacrylat-, Alkandioldimethacrylat- oder Cyanacrylatbasis verwendet. Das zur Füllung verwendete Glaspulver liegt in den fertigen Kunstharzpasten in Gewichtsanteilen von bis zu 80 Gew.-% vor, wobei angestrebt wird, den Glaspulveranteil aus Festigkeitsgründen so hoch wie nur eben möglich zu wählen.

### Beispiele:

Aus üblichen, reinen Rohstoffen wurden 6 Gläser erschmolzen, deren Zusammensetzung und Eigenschaften in der Tabelle zusammengefaßt sind. Für die physikalischen Eigenschaften werden der Brechwert für eine Wellenlänge von 587 nm (n_{d}), der Wärmeausdehnungskoeffizient α im Bereich von - 30 bis + 70 °C sowie die Al-Gleichwertdicke nach ISO 4049 bestimmt. Alle Gläser gehören der hydrolytischen Klasse 1 (nach ISO 719) an.

**Tabelle**

| | 1 | 2 | 3 | 4 | 5 | 6 |
|---|---|---|---|---|---|---|
| SiO₂ | 50.0 | 80.0 | 50.0 | 50.0 | 50.73 | 54.53 |
| B₂O₃ | 15.0 | 7.5 | 12.5 | 7.5 | 8.34 | 16.86 |
| Al₂O₃ | 15.0 | 7.5 | 10.0 | 15.0 | 8.99 | 7.93 |
| CaO | | | 2.5 | 6.0 | | |
| SrO | 20.0 | 25.0 | 25.0 | 21.5 | 31.94 | 19.83 |
| F₂-O | | | | | | 0.86 |
| F | | | | | | 1.48 |
| n_{d} | 1.53 | 1.53 | 1.55 | 1.46 | 1.56 | 1.51 |
| α₋₃₀₊₇₀ [·10⁻⁶/K] | 4.0 | 4.4 | 4.8 | 4.5 | 5.1 | 3.7 |
| Al-Gleichwertdicke | | | | | | |
| e. 2mm Glasprobe | 4.3 | 4.7 | 4.7 | 4.3 | 5.8 | 3.0 |

## Patentansprüche

1. Bariumfreies Dentalglas mit hoher Röntgenabsorption,
**gekennzeichnet durch**
eine Zusammensetzung (in Gew.-% auf Oxidbasis) von
| | |
|---|---|
| SiO₂ | 45 - 65 |
| B₂O₃ | 5 - 20 |
| Al₂O₃ | 5 - 20 |
| CaO | 0 - 10 |
| SrO | 15 - 35 |
| F₂-O | 0 - 2 |

2. Dentalglas nach Anspruch 1,
**gekennzeichnet durch**
eine Zusammensetzung (in Gew.-% auf Oxidbasis) von
| | |
|---|---|
| SiO₂ | 48 - 60 |
| B₂O₃ | 5 - 18,5 |
| Al₂O₃ | 5 - 18,5 |
| CaO | 0 - 7 |
| SrO | 17 - 35 |
| F₂-O | 0 - 2 |

3. Dentalglas nach Anspruch 1 oder 2,
**gekennzeichnet durch**
eine Zusammenfassung (in Gew.-% auf Oxidbasis) von
| | |
|---|---|
| SiO₂ | 50 - 56 |
| B₂O₃ | 6 - 15 |
| Al₂O₃ | 6 - 15 |
| CaO | 0 - 6 |
| SrO | 20 - 32 |
| F₂-O | 0 - 2 |

4. Dentalglas nach wenigstens einem der Ansprüche 1 - 3,
**gekennzeichnet durch**
einen Brechungsindex n_{d} ≦ 1.56, eine Aluminiumgleichwertdicke von ≧ 3 mm, insbesondere ≧4 mm und einen Wärmeausdehnungskoeffizienten α₋₃₀₊₇₀ ≦ 5 x 10⁻⁶/K, insbesondere <4,5 x 10⁻⁶/K

5. Verwendung eines Dentalglases nach wenigstens einem der Ansprüche 1 - 4 als Glaspulver mit einer mittleren Teilchengröße von < 10 µm, insbesondere von 0,5 bis 5 µm als Füllmittel für als Zahnfüllung verwendete Kunstharzpasten.
